# EUROPEAN PATENT SPECIFICATION

(11) **EP 3 061 448 B1**
(45) Date of publication and mention of the grant of the patent: **29.07.2020**
(21) Application number: 14854980.1
(22) Date of filing: 24.10.2014
(51) Int. Cl.: A61K 31/05, A61K 31/045, A61K 9/70, A61P 17/00, A61Q 19/00, A61K 9/00, A61K 9/06, A61P 17/04

(54) **COMPOSITION CONTAINING EUGENOL AS ACTIVE INGREDIENT FOR PREVENTING OR TREATING ATOPIC DERMATITIS**
ZUSAMMENSETZUNG MIT EUGENOL ALS WIRKSTOFF ZUR PRÄVENTION ODER BEHANDLUNG VON ATOPISCHER DERMATITIS
COMPOSITION CONTENANT DE L'EUGÉNOL COMME PRINCIPE ACTIF POUR PRÉVENIR OU TRAITER LA DERMATITE ATOPIQUE

(30) Priority: 24.10.2013 KR 20130127399; 06.08.2014 KR 20140101130
(43) Date of publication of application: 31.08.2016
(73) Proprietor: Seoul National University R&DB Foundation, Gwanak-gu Seoul 08826 (KR)
(72) Inventor: OH, Seog Bae, Seoul 136-753 (KR); JUNG, Sung Jun, Suwon-si Gyeonggi-do 440-709 (KR); CHUNG, Ge Hoon, Yongin-si Gyeonggi-do 448-737 (KR); KIM, Yong Ho, Seoul 136-731 (KR); LEE, Sang Hoon, Seoul 120-733 (KR)
(74) Representative: Zwicker, Jörk
(86) International application number: PCT/KR2014/010052
(87) International publication number: WO 2015/060677

(56) References cited:
- JP-A- 2004 091 477
- JP-B2- 3 905 267
- KR-A- 20060 092 373
- KR-A- 20080 006 837
- KR-A- 20120 044 396

## Description

### Technical Field

The present invention relates a pharmaceutical composition, a health functional food composition, a quasi-drug composition, and a cosmetic composition for preventing, treating, or improving atopic dermatitis, comprising eugenol or a derivative thereof, or a pharmaceutically acceptable salt thereof as an active ingredient.

### Background Art

Atopic dermatitis is a chronic, relapsing, itchy dermatitis. It is a disorder which often affects infants to children and is accompanied by pruritus, xeroderma, or specific eczema. Typical symptoms of atopic dermatitis are rash found in hands, scalp, face, neck, elbows, knees, etc., dry skin, pruritus, inflammation, and lichenification in which skin is peeled off as scales and thickens if scratched harshly, thereby forming deep wrinkles.

The direct cause of atopic dermatitis is still not clear, and thus research thereon has been continuously conducted. For treatment of atopic dermatitis, components from ceramide, linoleic acid, vegetable oil or mineral oil, steroid formulations such as hydrocortisone, or substances with antibacterial and anti-inflammatory functions added thereto have been suggested. However, the steroid formulations have a problem of inducing adverse effects such as epidermis growth inhibition, side effects, and hyperstimulation of the skin by urea peroxides, and side effects such as bacteria resistance, and photosensitivity at a high possibility. Further, prolonged administration on skin may induce severe side effects such as telangiectasia and/or keratin thickness increase and expansion. Gamma-linoleic acid, which is commonly used for alleviating atopic dermatitis, has low stability and comparably high skin irritation, thus not being suitable for sensitive skin.

Eugenol is a transparent pale yellow refined oil extracted from clove oil, nutmeg, cinnamon, basil, and bay leaf. It has a low solubility in water and high solubility in organic solvents. Also, as a spice, it has the fragrance of clove and may exhibit unpleasant scents when present at high concentration. Its name was also derived from the scientific names of cloves, i.e., *Eugenia aromaticum* or *Eugenia caryophyllata,* and eugenol consists of about 72% to 90% of refined oil extracted from cloves as a main ingredient. The antibacterial and anesthetic activities of eugenol have been known, and due to the ability of alleviating toothache typically induced by noxious heat stimuli, eugenol has been widely used in dentistry as a restoring agent and in prosthetic appliances by combining with zinc oxide [Markowitz, K. et al., OralSurg. OralMed. OralPathol., 1992, 73: 729-737]. However, nothing has been disclosed about effects of eugenol or a derivative thereof on pruritus.

### Disclosure

### Technical Problem

The present inventors aimed to develop a therapeutic agent for pruritus derived from natural products or novel compounds. As a result, the present inventors confirmed that eugenol or a derivative thereof may be used effectively for improving, preventing or treating pruritus, thereby completing the present invention.

### Technical Solution

An objective of the present invention is to provide a pharmaceutical composition, a health functional food composition, a quasi-drug composition, and a cosmetic composition for preventing, treating, or improving pruritus, comprising a compound represented by Formula 1 or 2 or a pharmaceutically acceptable salt thereof as an active ingredient, 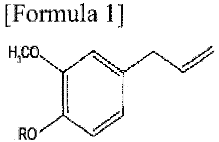 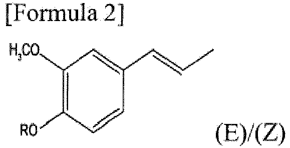 wherein, in the formulae, R is hydrogen or C₁₋₄ alkyl.

Another objective of the present invention is to provide a transdermal formulation for treating pruritus, comprising the pharmaceutical composition.

### Advantageous Effects

The compound of the present invention has an excellent antipruritic effect for the treatment of pruritus, and is nontoxic and therapeutically effective to overcome the side effects of the conventional pruritus therapeutic agents, and thus can be effectively used as a composition for preventing, treating, and improving pruritus.

### Description of Drawings

Fig. 1 illustrates the experimental procedure of producing atopic dermatitis animal models used in the present invention and measuring therapeutic activities of eugenol on atopic dermatitis.
Fig. 2 illustrates animal models of atopic dermatitis produced in the present invention and dermal recovery effects by eugenol treatment.
Fig. 3 illustrates an epidermal layer for confirming the use of eugenol in preventing or treating atopic dermatitis.
Fig. 4 illustrates the effects of eugenol on atopic pruritus.
Fig. 5 illustrates the therapeutic effects of eugenol on histamine-induced pruritus.
Fig. 6 illustrates the therapeutic effects of eugenol on serotonin-induced pruritus.

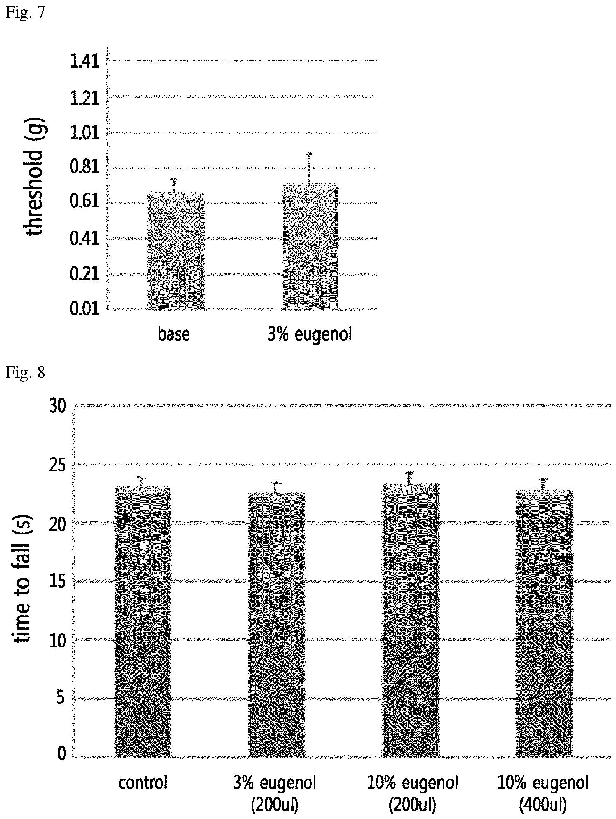

Fig. 7 illustrates the irrelevane of 3 wt% eugenol to therapeutic effects on mechanical pain.
Fig. 8 illustrates the effects of eugenol on reduced motor ability according to concentration and volume thereof. Specifically, it shows that 3 wt% eugenol specifically inhibits pruritus while not inducing a decrease in the motor ability of a subject.
Fig. 9 illustrates that 3 wt% eugenol inhibits capsaicin receptors, thereby increasing avoidance reactions to heat pain.
Fig. 10 illustrates that when a 3 wt% eugenol cream is administered on histamine-induced pruritus-affected areas, calming effects last up to 5 hours.
Fig. 11 illustrates the therapeutic effect of eugenol and a derivative thereof on histamine-induced pruritus.

### Best Mode

The present invention describes a pharmaceutical composition for preventing or treating atopic dermatitis, comprising a compound represented by Formula 1 or Formula 2 or a pharmaceutically acceptable salt thereof, 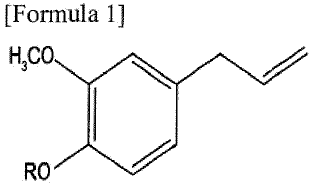 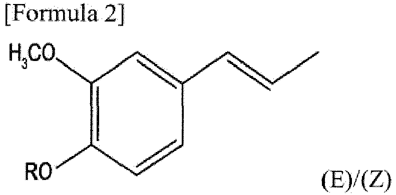 wherein, in the formulae, R is hydrogen or C₁₋₄ alkyl.

Particularly, the pharmaceutical composition for preventing or treating atopic dermatitis as described herein may comprise a compound represented by Formula 1 or Formula 2 or a pharmaceutically acceptable salt thereof

A compound represented by Formula 1 in which R is hydrogen is eugenol.

As used herein, the term "eugenol" refers to a transparent pale yellow refined oil extracted from clove oil, nutmeg, cinnamon, basil, and bay leaf, which consists of about 72% to 90% of refined oil extracted from cloves as a main ingredient. Eugenol is a compound of Formula 1 when R is hydrogen, thus having a structure represented by Formula 3, and is also known as 4-allyl-2-methoxyphenol.

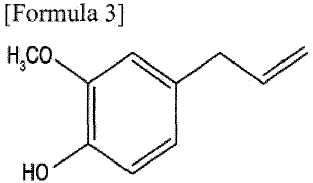

Since the antibacterial and anesthetic activities have been known about eugenol, eugenol has been widely used in dentistry as a restoring agent and in prosthetic appliances. However, due to its toxicity to liver, when used in a large amount, various symptoms of side effects such as hematuria, convulsions, diarrhea, nausea, unconsciousness, dizziness, and tachycardia., may be accompanied. The present invention confirmed that treating the eugenol to atopic dermatitis animal models results in recovery of epidermis pachynesis and significant alleviation of pruritus caused by atopic dermatitis.

As the eugenol is already used as a pain killer in dentistry, commercialized eugenol may be purchased and used, or eugenol may be extracted or refined from clove oil, nutmeg, cinnamon, basil, bay leaf, etc., or synthesized chemically using known methods in the art.

Further, R may be C₁₋₄ alkyl in a compound represented by Formula 1. Particularly, R may be methyl (CH₃-), ethyl (CH₃CH₂-), propyl (CH₃CH₂CH₂-), isopropyl (CH₃CHCH₃), *n*-butyl (CH₃CH₂CH₂CH₂-), *sec*-butyl (CH₃CHCH₂CH₃), *tert*-butyl ((CH₃)₃C-), etc., but is not limited thereto. The compounds may be named methyleugenol, ethyleugenol, propyleugenol, butyleugenol, etc., depending on the type of R. The method of producing the compounds may be (1) treating eugenol represented by Formula 3 with alkali, and (2) treating with halogenated alkyls, etc., but is not limited thereto.

A compound represented by Formula 2 in which R is hydrogen is isoeugenol.

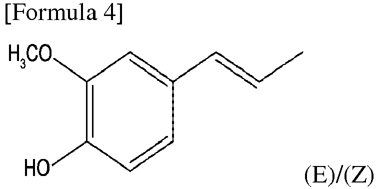

The IUPAC name of isoeugenol is (E) or (Z)-2-methoxy-4-(prop-1-enyl)phenol. Isoeugenol is a compound which is only different from eugenol represented by Formula 3 in the double bond location, and because the double bond is present in the center, it includes both (E) and (Z) structures. The method of producing the coumpound may include (1) treating eugenol with alkali in heat, and (2) treating acids, etc., but is not limited thereto.

Further, R may be C₁₋₄ alkyl in a compound represented by Formula 2. R may be methyl (CH₃-), ethyl (CH₃CH₂-), propyl (CH₃CH₂CH₂-), isopropyl (CH₃CHCH₃), *n*-butyl (CH₃CH₂CH₂CH₂-), *sec*-butyl (CH₃CHCH₂CH₃), *tert*-butyl ((CH₃)₃C-), etc., but is not limited thereto. The compounds may be named methylisoeugenol, ethylisoeugenol, propylisoeugenol, butylisoeugenol, etc., depending on the type of R. The method of producing the compounds may include (1) treating eugenol represented by Formula 4 with alkali, and (2) treating with halogenated alkyls, etc., but is not limited thereto.

As a derivative of the present invention, it may be a compound represented by Formula 5 or 6.

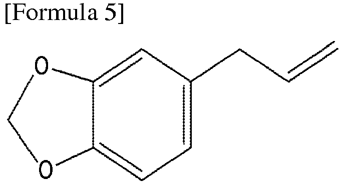

The compound is referred to as safrole or 5-allylbenzo[d][1,4]-dioxole, and the IUPAC name is 5-(2-propenyl)-1,3-benzodioxole. It is mainly used as a synthetic material for spices, piperonal or vanillin, and is also used as analgesic ointments for rheumatoid arthritis. Safrole, as a derivative of eugenol, may be synthesized by the following method, but is not limited thereto:

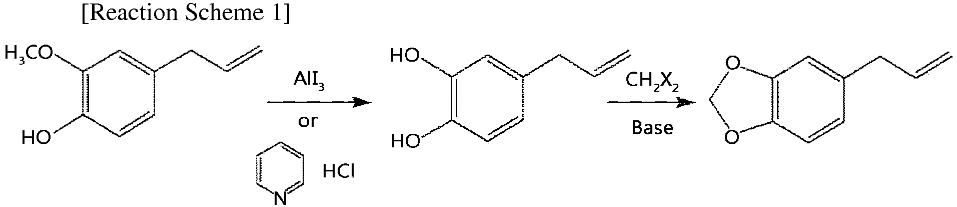

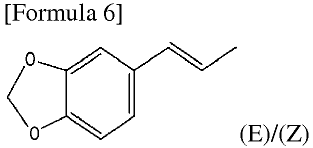

The compound is referred to as isosafrole, and the IUPAC name is (E) or (Z)-5-(prop-1-enyl)benzo[d][1,3]-dioxole. Isosafrole, as a derivative of isoeugenol, has a double bond in the center, and thus may include both (E) and (Z) structures. Isosafrole, as a derivative of isoeugenol, may be synthesized by the following method, but is not limited thereto:

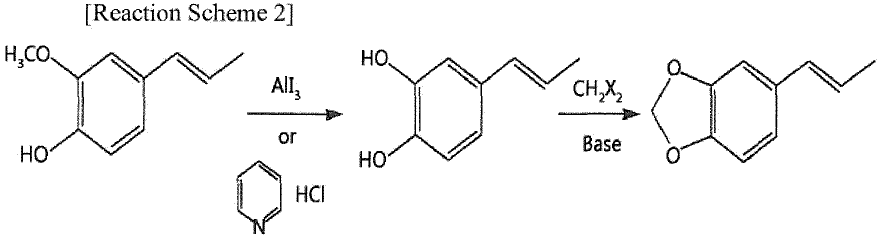

As a pharmaceutically acceptable salt of the compound, an acid addition salt formed by a pharmaceutically acceptable free acid may be useful. As a free acid, an organic acid and an inorganic acid may be used. As an inorganic acid, hydrochloric acid, hydrobromic acid, sulfuric acid, sulfurous acid, phosphoric acid, etc., may be used, and as an organic acid, citric acid, acetic acid, maleic acid, fumaric acid, gluconic acid,methane sulfonic acidglycolic acid, succinic acid, tartaric acid, 4-toluene sulfonic acid, galacturonic acid, embonic acid, glutamic acid, aspartic acid, etc., may be used.

Addition salts of the present invention may be produced by conventional methods such as dissolving the compound in a water-miscible organic solvent such as acetone, methanol, ethanol, acetonitrile, etc., adding an equivalent of or an excessive organic acid or an acid aqueous solution of an inorganic acid, followed by precipitation or crystallization; or by evaporating a solvent or excessive acid followed by drying or suction filtration of precipitated salts.

The present invention may comprise not only the compound or a pharmaceutically acceptable salt thereof, but also solvates, hydrates, and stereoisomers that may be produced therefrom, having equivalent effects within the scope of the present invention.

In one embodiment, the present invention confirmed that eugenol or representative derivatives thereof, methyleugenol and isoeugenol, help recovery of epidermis pachynesis in atopic dermatitis animal models, and significantly alleviates pruritus caused by atopic dermatitis as well, thereby confirming that they may be effectively used for improving, preventing or treating pruritus. Therefore, it is obvious that the compounds represented by Formula 1 or Formula 2 have the effects of improving, preventing or treating pruritus.

As used herein, the term "preventing" refers to any activity that inhibits or delays occurrence of pruritus by administering the composition of the present invention.

As used herein, the term "treating" refers to any activity that alleviates or positively changes symptoms of pruritus by the composition of the present invention.

As used herein, the term "atopic dermatitis" refers to a chronic, relapsing inflammatory dermatitis accompanied by pruritus, xeroderma, or specific eczema. A significant increase in serum IgE is characteristically found in acute lesions of atopic dermatitis. Further, atopic dermatitis may be diagnosed and the severity may be examined via histopathological changes in lesions and sensory tests to affected lesions, etc. The cause of atopic dermatitis has not been fully understood yet, but it has been suggested that genetics along with immunological and non-immunological pathways are involved.

Preferably, a compound represented by Formula 1 or Formula 2 or a pharmaceutically acceptable salt thereof of the present invention is capable of preventing or treating atopic dermatitis by inhibiting or blocking capsaicin receptors. Preferably, the capsaicin receptors are transient receptor potential cation channel subfamily V member 1 (TRPV1), which are often expressed in pain-signaling peripheral nerves and are known to play a critical role in the sense of pain. Eugenol not only has the effects of blocking voltage-dependent Na ion channels, it may act on and activate TRPV1 at high concentration of 1 mM or more. Therefore, it is known to alleviate pain by activating capsaicin receptors. However, the present invention confirmed that eugenol at a low concentration of 3 wt%, on the contrary, blocks capsaicin receptors and treats histamine-dependent pruritus caused by atopic dermatitis.

A pharmaceutical composition comprising a compound represented by Formula 1 or Formula 2 or a pharmaceutically acceptable salt thereof of the present invention may further include an appropriate carrier, excipient, or diluent which are conventionally used in production of a pharmaceutical composition. Here, the compound represented by Formula 1 or Formula 2 or a pharmaceutically acceptable salt thereof may be comprised in the composition in the amount of 0.01 wt% to 50.0 wt%, preferably 2 wt% to 20 wt%, and more preferably 3 wt% to 10 wt% with respect to the total composition weight, but is not limited thereto.

The pharmaceutical composition may be in any form selected from the group consisting of tablets, pills, powders, granules, capsules, suspensions, solutions, emulsions, syrups, sterile aqueous solutions, nonaqueous solvents, freeze-drying agents, and suppositories, and may be various oral or parenteral formulations. When formulated, it is formulated using typically used diluents or excipients, such as fillers, extenders, binders, wetting agents, disintegrating agents, surfactants, etc. The solid formulation for oral administration includes tablets, pills, powders, granules, capsules, etc., and is prepared by mixing at least one excipient such as starch, calcium carboante, sucrose or lactose, gelatin, etc. Further, besides simple excipients, lubricants such as magnesium stearate, talc, etc., may be also used. The liquid formulation for oral administration may include suspensions, solutions, emulsions, syrups, etc., and may also include various excipients such as wetting agents, flavors, fragrances, preservatives, etc., besides simple diluents such as water and liquid parabens. The formulation for parenteral administration may include sterile aqueous solutions, nonaqueous solvents, suspensions, emulsions, freeze drying agents and suppositories. As nonaqueous solvents and suspension formulations, propylene glycol, polyethylene glycol, vegetable oil such as olive oil, injectable esters such as ethyloleate, etc, may be used. As the base for suppositories, witepsol, macrogol, tween 61, cacao butter, laurin butter, glycerogelatin, etc., may be used.

The pharmaceutical composition of the present invention may be administered at a pharmaceutically effective amount.

As used herein, the term "pharmaceutically effective amount" refers to a sufficient amount to treat or prevent diseases, at a reasonable benefit/risk ratio applicable to any medical treatment or prevention. The effective dosage level may be determined depending on a subject's type, severity of a disease, age, and sex, a type of a disease, activity of the drug, sensitivity to the drug, administration time, administration route and excretion rate, duration of treatment, drugs used simultaneously with the composition of the present invention, and other factors known in the medical field. The pharmaceutical composition of the present invention may be administered alone or in combination with other therapeutic agents, and may be administered sequentially or simultaneously with conventional therapeutic agents. The composition may be administered in a single or multiple dosage form. It is important to administer the composition in the minimum amount that can exhibit the maximum effect without causing side effects, in consideration of all the above factors, and the amount may be easily determined by one of ordinary skill in the art.

The preferred administration amount of the composition of the present invention may differ depending on a patient's condition and weight, severity of a disease, form of a drug, and administrative route and duration. The suitable daily dosage may be determined by one who performs treatments within the scope of appropriate medical decisions, and may be typically 0.001 mg/kg to 1000 mg/kg, preferably 0.05 mg/kg to 200 mg/kg, and more preferably 0.1 mg/kg to 100 mg/kg administered once or in a few divided doses a day. The composition may be administered to any subject as long as the purpose is to prevent or treat pruritus, without particular limitations. For example, the composition may be applied to any subjects human or nonhuman animals such as monkeys, dogs, cats, rabbits, marmots, rats, mice, cows, sheep, pigs, and goats, etc. The administrative routes include any methods as long as it is a conventional method in the art. For example, it may be a dermal administration method such as topical application, but is not limited thereto.

In one embodiment, the present invention confirmed that among atopic dermatitis animal models induced by applying oxazolone compounds, experimental groups treated with 3 wt% to 10 wt% eugenol or a derivative thereof showed recovered epidermis pachynesis and alleviated pruritus caused by atopic dermatitis (Experimental Examples 1 to 3). This demonstrates that 3 wt% to 10 wt% eugenol is effective in treating pruritus.

Further, the present invention provides a transdermal formulation for treating pruritus comprising the pharmaceutical composition of the present invention.

Particularly, by formulating the pharmaceutical composition for dermal administration, the composition may be used for treating pruritus.

As used herein, the term "a transdermal formulation" refers to a formulation or preparation for dermal application or administration, which shows effects of administering drugs through skin and is formulated into a drug for applying on skin, for attaching to skin, etc. During dermal administration, skin penetration of an active ingredient takes place through intracellular, intercellular or appendages such as sweat pores, hair pores, due to chemical potential, for example, diffusion by the concentration gradient.

There exists a disadvantage of having difficulty in penetrating undamaged skin, but there also exist easiness in usage such as effectiveness of a drug, control of administration speed, direct application on the affected areas, etc., and advantages of maintaining comparably stable concentration in blood, minimizing side effects of substances toxic to the gastrointestinal tract, reducing toxicity on the liver, etc. In order to enable easy penetration of an active ingredient, skin penetrating promoters may be additionally included and formulated. The composition of the present invention is lipid-soluble and may be advantageous in dermal administration.

The transdermal formulation of the present invention is not particularly limited and may be any formulation as long as it can directly apply active ingredients on the surface of affected skin areas. For example, a formulation may be prepared in the form of ointments, creams, gels, lotions, solutions, emulsions, suspensions, sticks, pastes, liniments, cataplasms, tapes, aerosols, external powders, etc. Suitable carriers, excipients, and diluents, conventionally used in the preparation of the ormulation for dermal application, may be further included.

Formulations in the form of ointments, creams, gels, and lotions may include bases such as white petrolatum, yellow petrolatum, lanolin, bleached beeswax, cetanol, stearyl alcohol, stearic acid, hydrogenated oil, gelated hydrocarbon, polyethylene glycol, liquid paraffin, squalane, etc.; solvents and solubilizers such as oleic acid, myristic acid isopropyl, tri iso-octane acid glycerin, crotamiton, sebacic acid diethyl, diisopropyl adipate, hexyl laurate, fatty acids, fatty acid esters, aliphatic alcohols, vegetable oil, etc; antioxidants such as tocopherol derivatives, L-ascorbic acids, dibutyl hydroxy toluene, butyl hydroxy anisole, etc.; preservatives such as parahydroxybenzoic acid esters, etc.; moisturizers such as glycerin, propylene glycol, sodium hyaluronate, etc.; surfactants such as polyoxyethylene derivatives, glycerin fatty acid esters, sucrose fatty acid esters, sorbitan fatty acid esters, propylene glycol fatty acid esters, lecithin, etc.; and thickeners such as carboxyvinyl polymer, xanthan gum, carboxymethyl cellulose, carboxymethyl cellulose sodium salt, hydroxypropyl cellulose, hydroxypropyl methyl cellulose, etc. Further, stabilizers, preservatives, absorption promoters, pH adjusting agents, and other suitable additives may be added as desired.

Formulations in the form of solutions or emulsions may include solvents, emulsifying agents or demulsifying agents such as water, ethanol, isopropanol, ethyl carbonate, ethyl acetate, benzyl alcohol, benzyl benzoate, propylene glycol, 1,3-butylglycol oil, glycerol aliphatic esters, polyethylene glycol, or sorbitan fatty acid esters as carrier substances, etc.

Formulations in the form of suspensions may include, as carrier substances, liquid diluents such as water, ethanol, or propylene glycol; suspensions such as ethoxylated isosteatryl alcohol, polyoxyehtylene sorbitol esters and polyoxyethylene sorbitan esters; and microcrystalline cellulose, aluminum metahydroxide, bentonite, agar, tragacanth, etc.

Formulations in the form of sticks may be formulated using lead monoxide, olive oil, and lard. Formulation in the form of pastes may be formulated using zinc cemal, salicylic acid cemal, starch and petrolatum. Formulations in the form of liniments may be formulated using camphor oil, olive oil, methyl salicylate, tragacanth, sodium carboxymethyl cellulose, glycerol, and zinc oxide.

Formulations in the form of cataplasm may include tackifiers such as polyacrylic acid, polyacrylic acid copolymers, etc.; cross-linking agents such as aluminum sulfate, potassium aluminum sulfate, aluminum chloride, magnesium metasilicate aluminate, dihydroxy aluminum acetate, etc.; thickeners such as sodium polyacrylate, polyvinylalcohol, Polyvinylpyrrolidone, gelatin, sodium alginate, carboxymethyl cellulose, carboxymethyl cellulose sodium salts, hydroxypropyl cellulose, hydroxypropyl methyl cellulose, etc.; polyalcohols such as glycerin, polyethylene glycol (macrogol), propylene glycol, 1,3-butanediol, etc.; surfactants such as polyoxyehtylene derivaties, etc; fragrances such as 1-methol, etc.; preservatives such as parahydroxybenzoic acid esters, etc.; purified water; etc. Further, stabilizers, preservatives, absorption promoters, pH adjusting agents, and other suitable additives may be added as desired.

Formulations in the form of tapes may include adhesives such as styrene, isoprene, styrene block copolymers (SIS block copolymers), acryl resins, etc.; adhesive resins such as alicyclic saturated hydrocarbon resins, rosin resins, terpene resins, etc.; softeners such as liquid rubbers, liquid paraffins, etc.; antioxidants such as dibutyl hydroxy toluene, etc.; polyalcohol such as propylene glycol, etc.; absorption promoters such as oleic acids; surfactants such as polyoxyehtylene derivatives, etc., and other suitable additives. Further, by adding polymers capable of containing water, such as sodium polyacrylate or polyvinyl alcohol, and a small amount of purified water, it may be in the form of water-containing tapes. In such case, stabilizers, preservatives, absorption promoters, pH adjusting agents, and other suitable additives may also be further added as desired.

Formulations in the form of aerosols may include bases such as white petrolatum, yellow petrolatum, lanolin, bleached beeswax, cetanol, stearyl alcohol, stearic acid, hydrogenated oil, gelated hydrocarbon, polyethyleneglycol, liquid paraffin, squalane, etc. used in preparing ointments, creams, gels, suspensions, emulsions, solutions and lotions; solvents and solubilizers such as oleic acid, lauric acid isopropyl, isopropyl adipate, sebacic acid isopropyl, tri iso-octane acid glycerin, crotamiton, sebacic acid diethyl, hexyl laurate, fatty acids, fatty acid esters, aliphatic alcohol, vegetable oil etc.; antioxidants such as tocopherol derivative, L-ascorbic acid, dibutyl hydroxy toluene, butyl hydroxy anisole, etc.; preservatives such as para-hydroxy benzoic acid esters, etc.; moisturizers such as glycerin, propylene glycol, sodium hyaluronate, etc.; surfactants such as polyoxyehtylene derivatives, glycerin fatty acid esters, sucrose fatty acid esters, sorbitan fatty acid esters, propylene glycol fatty acid esters, lecithin, etc.; thickeners such as carboxyvinyl polymer, xanthan gum, carboxymethyl cellulose, carboxymethyl cellulose sodium salts, hydroxypropyl cellulose, hydroxypropyl methyl cellulose, etc. various stabilizers, buffers, flavorings, suspending agents, emulsifiers, fragrances, preservatives, solubilizers, and other suitable additives may be added. Especially, propellants such as chlorofluorohydrocarbon, propane/butane or dimethyl ethers may be further included.

Formulations in the form of external powders may include excipients such as potato starch, rice starch, corn starch, talc, zinc oxide, etc., or other suitable additives. In such case, stabilizers, preservatives, absorption promoters, pH adjusting agents, and other suitable additives may also be further added as desired.

The method of preparing a transderamal formulation provided in the present invention is not particularly limited, and may be prepared by conventional methods of preparing a transderaml formulation, such as sufficiently kneading each substance and base substances as needed according to the desired form, etc. Additionally, it may be prepared in the form of cataplasms and tapes by flattening the kneaded mixture onto release paper, drying, further attaching to flexible support, and cutting into desired sizes.

When the transderaml formulation provided in the present invention is in the form of ointments, liquids (suspension, emulsions, lotions, etc.), aerosols, and external powders, it may be used by conventional methods such as directly applying such as applying to the affected skin areas, etc., or indirectly applying such as applying on supports such as clothes, etc., and putting on skin, etc. Further, when the formulation is in the form of cataplasms or tapes, it may be directly applied to the affected skin areas.

The applied dosage of the transderaml formulation of the present invention may be determined considering percutaneous penetration speed, cell absorption ability, etc. Preferably, dosage per skin unit area of 1 mg/cm² to 50 mg/cm² or 2 mg/cm² to 20 mg/cm² may be advised, but is not limited thereto, and may be determined by one of ordinary skill in the art in consideration of the content of a compound represented by Formula 1 or Formula 2 or a pharmaceutically acceptable salt thereof to the total composition weight. A compound represented by Formula 1 or Formula 2 or a pharmaceutically acceptable salt thereof of the transdermal formulation of the present invention may be prepared at 3 wt% to 10 wt% with respect to the total composition weight, but is not limited thereto, and may be determined by one of ordinary skill in the art in the scope of providing effects of a compound represented by Formula 1 or Formula 2 or a pharmaceutically acceptable salt thereof on improving, preventing or treating pruritus.

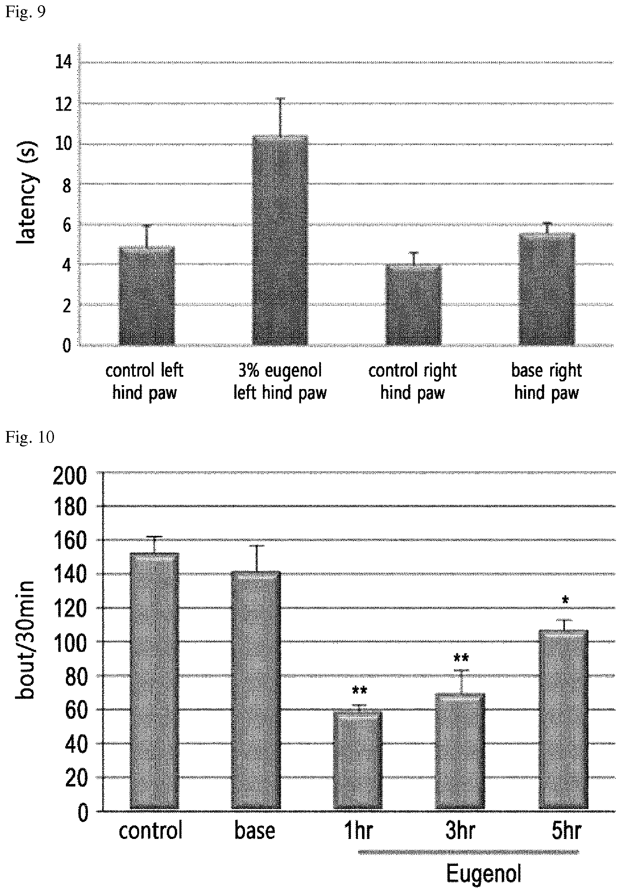

In one embodiment of the present invention, it was confirmed that when the transdermal formulation containing 3 wt% eugenol is applied on the histamine-induced pruritus affected areas, calming effects last up to 5 hours (Experimental Example 8 and Fig. 10).

The present invention provides a composition for preventing, treating, or improving pruritus, comprising the compound represented by Formula 1 or Formula 2 or a pharmaceutically acceptable salt thereof as an active ingredient, 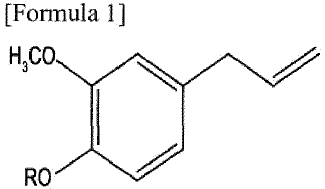 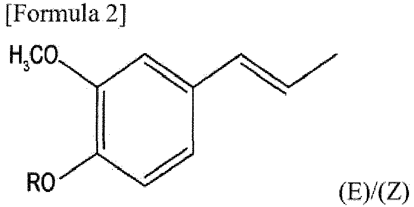 wherein, in the formulae, R is hydrogen or C₁₋₄ alkyl.

Particularly, the compound or a pharmaceutically acceptable salt thereof may be used for preventing, treating, or improving pruritus, and the composition may comprise a pharmaceutical composition, a health functional food composition, a quasi-drug composition, or a cosmetic composition.

As used herein, the term "pruritus" refers to a general symptom of the skin condition inducing the inclination to scratch and displeasure and although it may generally take place irrespective of external stimuli, it may also be induced by stimuli such as light contact, temperature change, stress, etc., and chemical, physical, and electric stimuli. Pruritus is a disorder generally accompanied in the case of various skin disorders or systemic diseases, and includes both acute and chronic pruritus.

Skin disorders accompanying pruritus, which are subjected to treatment, may be atopic dermatitis, neurodermatitis, contact dermatitis, seborrheic dermatitis, self-sensitization dermatitis, caterpillar dermatitis, sebum deficiency, senile skin pruritus, skin infection from insect bite, photophyersensitivity, urticaria, prurigo, herpes, impetigo, eczema, tinea, lichen, psoriasis, acne vulgaris, scabies, hives, etc. Further, systemic disease accompanying pruritus may be diabetes, chronic renal failure, chronic hemodialysis, biliary obstructive disorders, anemia, hematologic malignancy (leukemia, polycythemia mellitus, and Hodgkin's lymphoma), increased intestinal parasites, hyperthyroidism, hypothyroidism, AIDS, etc.

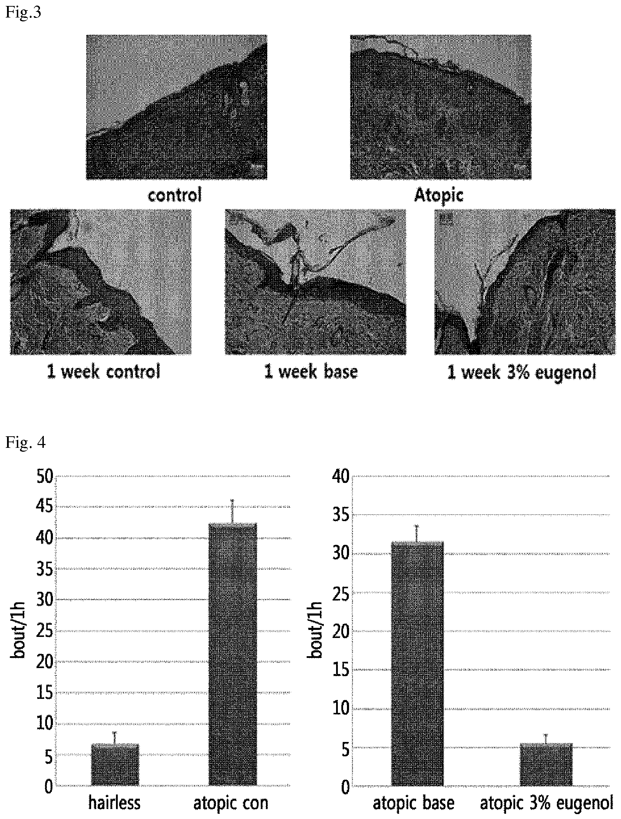

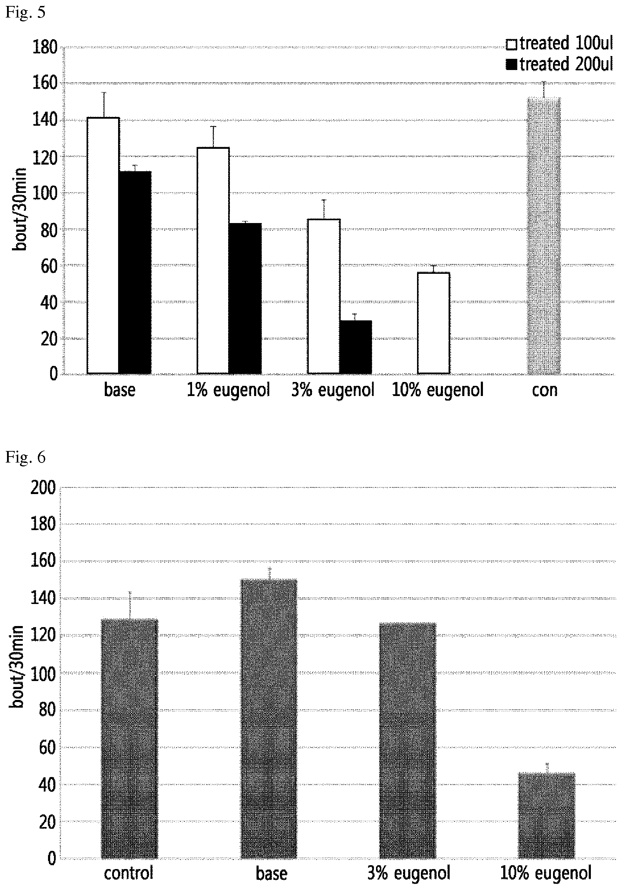

In one embodiment of the present invention, it was confirmed that when eugenol and representative derivatives thereof, methyleugenol and isoeugenol, were each administered, histamine-induced pruritus was significantly reduced. Therefore, it is obvious that the compound represented by Formula 1 or Formula 2 may be comprised as an active ingredient of a composition for preventing, treating, or improving pruritus (Experimental Examples 3 to 5, Figs. 4 to 6, and 11).

Further, the present invention provides an antipruritic agent comprising a compound represented by Formula 1 or Formula 2 or a pharmaceutically acceptable salt thereof as an active ingredient, 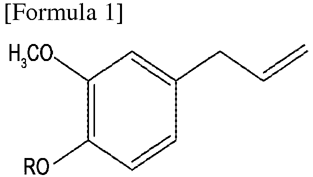 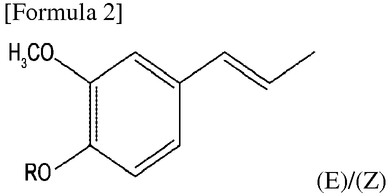 wherein, in the formulae, R is hydrogen or C₁₋₄ alkyl.

Particularly, the compound, or a pharmaceutically acceptable salt thereof may be used for treating pruritus.

As described, when eugenol or representative derivatives thereof, methyleugenol and isoeugenol, of the present invention are each administered, antipruritic effects are confirmed. Therefore, it is obvious that they may be comprised as an active ingredient of an antipruritic agent.

Further, the present invention describes a health functional food composition for preventing or improving atopic dermatitis, comprising a compound represented by Formula 1 or Formula 2 or a pharmaceutically acceptable salt thereof as an active ingredient, 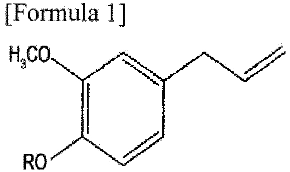 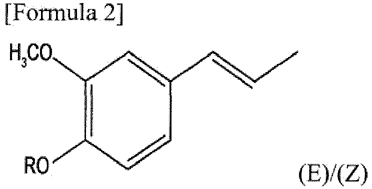 wherein, in the formulae, R is hydrogen or C₁₋₄ alkyl.

Particularly, the compound represented by Formula 1 or Formula 2 or a pharmaceutically acceptable salt thereof of the present invention may be comprised in a health functional food composition for preventing or improving atopic dermatitis as described herein.

The compound, the pharmaceutically acceptable salt, and atopic dermatitis are the same as described above.

As used herein, the term "improving" refers to any activity that alleviates or ameliorates symptoms of subjects suspected of or having atopic dermatitis as described herein using the composition of the present invention.

When the composition of the present invention is comprised and used in health functional food, the composition may be added alone or with health functional food or health functional food substances, and may be appropriately used according to conventional methods. The content ratio of active ingredients may be appropriately determined depending on the purpose of the use. Generally, when producing food or drinks, a composition of the present invention may be added at preferably up to 15 parts by weight, and more preferably up to 10 part by weight with respect to a raw material. However, in the case of long-period consumptions for the purpose of health control and sanitation, the amount may be lower than the suggested range. Also, because it is not prone to safety problems, the active ingredients may be used above than the suggested range.

The types of health functional food comprising a composition of the present invention is not particularly limited, and specific examples thereof are meats, sausages, bread, chocolate, candies, snacks, sweets, pizza, ramen noodles, other noodles, gums, dairy products including ice cream, soups, beverages, tea, drinks, alcoholic drinks, vitamin complex, etc. Conventionally understood health function food may be all included, and food used as feed for animals may be also included.

Further, when a health functional food composition of the present invention is used in the form of drinks, it may include additional components such as sweeteners, flavorings, natural carbohydrates, etc., like other beverages. The natural carbohydrates may be monosaccharides such as glucose and fructose, maltose, disaccharides such as sucrose, dextrin, polysaccharides such as cyclodextrins, and sugar alcohols such as xylitol, sorbitol, and erythritol. The ratio of the natural carbohydrates is not limited thereto, but may be preferably in the range of from about 0.01 g to about 0.04 g and more preferably about 0.02 g to about 0.03 g per 100 ml of a composition of the present invention. The sweetener may be natural sweeteners such as thaumatin and stevia extracts, and artificial sweeteners such as saccharin and aspartame.

A health functional food composition of the present invention may further contain various nutrients, vitamins, electrolytes, flavorings, colorings, pectic acid and its salts, alginic acid and its salts, organic acid, protective colloidal thickeners, pH buffers, stabilizers, preservatives, glycerin, alcohol, carbonating agents used in carbonated drinks, etc. Furthermore, fruit flesh used for producing natural fruit juice, fruit juice drinks, and vegetable drinks may be contained.

Further, the present invention describes a quasi-drug composition for preventing or improving atopic dermatitis, comprising a compound represented by Formula 1 or Formula 2 or a pharmaceutically acceptable salt thereof as an active ingredient, 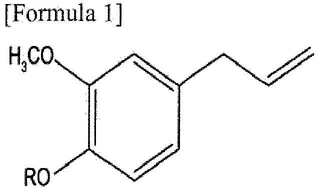 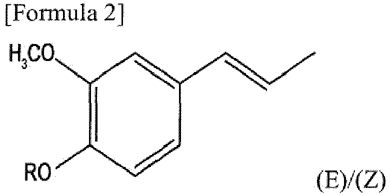 wherein, in the formulae, R is hydrogen or C₁₋₄ alkyl.

Particularly, the compound of the present invention or a pharmaceutically acceptable salt thereof may be comprised in a quasi-drug composition for preventing or improving atopic dermatitis as described herein.

The compound, the pharmaceutically acceptable salt, and atopic dermatitis are the same as described above.

As used herein, the term "a quasi-drug" refers to over-the-counter(OTC) drugs, specifically an article corresponding to one of fibers, rubber products, or the like used to treat, alleviate, eliminate, or prevent a disease of humans or animals; articles of which mechanisms are weak to human body or do not work directly on human body, and which are not apparatus or machines, or the like; and formulations used to sterilize, insecticide, or the like for preventing infection. Further, it refers to articles for diagnosing, treating, alleviating, eliminating, or preventing a disease of humans or animals, excluding apparatus, machines, or equipment; and articles for pharmaceutically effecting the organization and function of humans or animals, excluding apparatus, machines, or equipment. It also includes skin externals and personal care articles.

When the composition of the present invention is comprised in a quasi-drug for preventing or improving atopic dermatitis as described herein, the composition may be comprised alone or in combination with other quasi-drug components, and may be appropriately used following conventional methods. The content of active ingredients may be appropriately determined according to the purpose of the usage.

The topical dermatologic drugs are not particularly limited thereto, but may be prepared and used in the form of, for example, ointments, lotions, sprays, patches, creams, powders, suspensions or gel.

Further, the present invention describes a cosmetic composition for preventing or improving atopic dermatitis, comprising a compound represented by Formula 1 or Formula 2 or a pharmaceutically acceptable salt thereof as an active ingredient, 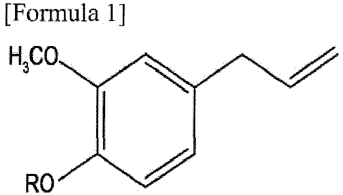 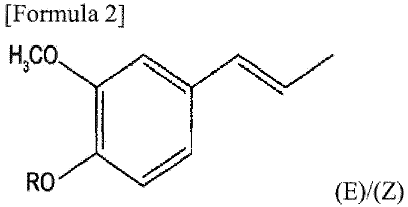 wherein, in the formulae, R is hydrogen or C₁₋₄ alkyl.

Particularly, the compound of the present invention or a pharmaceutically acceptable salt thereof may be comprised in a cosmetic composition for preventing or improving atopic dermatitis as described herein.

The compound, the pharmaceutically acceptable salt, and atopic dermatitis are the same as described above.

The cosmetic composition for preventing or improving atopic dermatitis described herein may contain the compound or a pharmaceutically acceptable salt thereof at 0.001 wt% to 50 wt%, more preferably 0.01 wt% to 20 wt%, and most preferably 3 wt% to 10 wt% with respect to the total composition weight, but is not limited thereto.

Further, a cosmetic composition of the present invention may contain other substances which are conventionally accepted, besides the mentioned active ingredients, without limitations. For example, it may include antioxidants, stabilizers, solubilizers, vitamins, and conventional adjuvants, such as pigments and fragrances, and carriers.

A cosmetic composition of the present invention may be prepared in the form of solutions, topical ointments, creams, foams, nutritious skin lotions, softening skin lotions, masks, softeners, liquids, makeup bases, essences, soaps, liquid soap, bath preparations, sun screen creams, sun oil, suspensions, emulsions, pastes, gels, lotions, powders, soaps, surfactant-containing cleansing, oil, powder foundations, emulsion foundations, wax foundations, patches, and sprays, but is not limited thereto.

Further, a cosmetic composition of the present invention may additionally contain at least one kind of a cosmetically acceptable carrier which is normally mixed in skin cosmetics. For example, conventional substances such as oil, water, surfactants, humectants, low alcohols, thickeners, chelating agents, dyes, preservatives, fragrances, etc., may be appropriately mixed, but are not limited thereto. A cosmetically acceptable carrier contained in a cosmetic composition of the present invention may vary depending on a formulation.

When a formulation of the present invention is an ointment, a paste, a cream, or gel, animal oil, vegetable oil, waxes, paraffins, starch, tragacanth, cellulose derivatives, polyethylene glycol, silicon, bentonite, silica, talc, zinc oxide, or a mixture thereof may be used as a carrier component.

When a formulation of the present invention is a powder or spray, actose, talc, silica, aluminum hydroxide, calcium silicate, polyamide powders, or mixtures thereof may be used as a carrier component. Especially when the formulation is a spray, it may further contain propellants such as chlorofluorohydrocarbon, propane/butane, or dimethyl ether.

When a formulation of the present invention is a solution or emulsion, solvents, solubilizers or demulsifying agents may be used as a carrier component. For example, water, ethanol, isopropanol, ethyl carbonate, ethyl acetate, benzyl alcohol, benzyl benzoate, propylene glycol, 1,3- butyl glycol oil may be used, and in particular, cottonseed oil, peanut oil, corn germ oil, olive oil , castor oil, sesame oil, glycerol aliphatic esters, polyethylene glycol, or sorbitan fatty acid esters may be used.

When a formulation of the present invention is a suspension, liquid diluents such as water, ethanol, or propylene glycol, suspensions such as ethoxylated isosteatryl alcohol, polyoxyehtylene sorbitol esters, and polyoxyehtylene sorbitan esters, microcrystalline cellulose, aluminum meta-hydroxide, bentonite, agar or tragacanth, etc., may be used as a carrier component.

When a formulation of the present invention is soap, alkali metal salts, fatty acids hemiesters salts, fatty acid protein hydrolysates, isethionates, lanolin derivatives, aliphatic alcohols, vegetable oil, glycerol, sugars, etc., may be used as a carrier component.

When a formulation of the present invention is surfactant-containing cleansing, aliphatic alcohol sulfate, aliphatic alcohol ether sulfates, mono sulfosuccinate esters, isethionates, imidazolinium derivatives, methyl taurates, sarcosinate, fatty acid amide ether sulfates, alkyl amido betaine, aliphatic alcohol, fatty acid glycerides, fatty acid diethanol amide, vegetable oil, lanolin derivatives, or ethoxylated glycerol fatty acid esters, etc., may be used as a carrier component.

Further, the present invention describes a method for preventing or treating atopic dermatitis comprising administering the pharmaceutical composition to a subject suspected of atopic dermatitis.

A subject suspected of atopic dermatitis as described herein refers to animals including humans, which have atopic dermatitis or potential of having atopic dermatitis. By administering a pharmaceutical composition comprising the compound of the present invention or a pharmaceutically acceptable salt thereof to a subject suspected of atopic dermatitis as described herein, the subject may be efficiently treated. Atopic dermatitis is the same as described above.

As used herein, the term "administering" refers to introducing a pharmaceutical composition of the present invention to a subject suspected of atopic dermatitis as described herein via appropriate methods. Administration routes may be various oral or parenteral routes that can deliver the composition to desired tissues, and may particularly be percutaneous administration via topical application, etc., but are not limited thereto.

The present invention may comprise administering a pharmaceutical composition comprising the compound represented by Formula 1 or Formula 2 or a pharmaceutically acceptable salt thereof at a pharmaceutically effective dose. The suitable daily dosage may be determined by one who performs treatments within the scope of appropriate medical decisions, and may be typically 0.001 mg/kg to 1000 mg/kg, preferably 0.05 mg/kg to 200 mg/kg, and more preferably 0.1 mg/kg to 100 mg/kg administered once or in a few divided doses a day. However, according to the objectives of the present invention, the effective dose of a particular patient should be preferably differently applied depending on the type and extent of a desired response, a formation which differs according to conditions, a particular composition, a subject's age, weight, general health condition, sex, and diet, administration time, administration route and excretion rate, duration of treatment, drugs used simultaneously or in combination with the particular composition, and other factors known in the medical field.

Further, the present invention describes an use of a composition comprising the compound represented by Formula 1 or Formula 2, or a pharmaceutically acceptable salt thereof as an active ingredient, or of a transderamal formulation for preventing or treating atopic dermatitis.

Further, the present invention describes an use of a composition comprising the compound represented by Formula 1 or Formula 2, or a pharmaceutically acceptable salt thereof as an active ingredient, or of a transdermal formulation for preparation or manufacture for preventing or treating atopic dermatitis.

### Mode for Invention

Hereinafter, the present invention will be described more in detail with reference to Examples, but Examples are for illustrative purposes only, and thus the scope of the present invention is not intended to be limited by the Examples.

### Preparation Example: Production of eugenol cream

Firstly, for oil phase preparation, eugenol, IPM, liquid paraffin, cetostearyl alcohol, glyceryl monostearate, Brij 58, Span 60, BHA, and methyl paraben were combined and heated for the mixture to reach 70°C to 80°C, thereby uniformly dissolving the mixture. Also, for liquid phase preparation, PEG1500, PEG4000, SLS, xanthan gum, and purified water were combined and dissolved at 70°C to 80°C. Thereafter, in order to mix the oil phase product and liquid phase product, they were stirred for 20 minutes at 70°C to 80°C, frozen, and mixed well with purified water, thereby producing a cream formulation at room temperature.

Eugenol as an active ingredient, was added at 3 different concentrations (1 wt%, 3 wt%, and 10 wt%) and was emulsified components following the substances and contents described in Table 1, followed by degassing, filtering, and cooling, which produced a cream formulation. For the control cream formulation, a dermal cream formulation was prepared in the same manner except that eugenol was not comprised. It was confirmed that the formulations have excellent formulation stability and did not have dermal side effects.

**Table 1**

| Object | Ingredients | Content (g/kg) |
|---|---|---|
| Base | IPM | 70 |
| | Liquid paraffin | 40 |
| | Cetostearyl alcohol | 30 |
| | Glyceryl monostearate | 30 |
| | PEG 1500 | 30 |
| | PEG 4000 | 30 |
| Emulsifier | Brij 58 | 15 |
| | Span 60 | 30 |
| | SLS | 2 |
| Stabilizer | BHA | 1 |
| Preservative | Methyl paraben | 1.8 |
| Solvent | Purified water | Suitable amount |

### Example: Inducing atopic dermatitis by treating oxalozone

7-week-old male C57/BL6 mice were used in the present experiment, and in order to produce atopic dermatitis models, 7-week-old male balb/c nude mice were purchased from Orient Bio (Korea) and used. The method described in the manuscript of Kuraishi et al., presented the original form of mouse models developed in the present invention (Kuraishi et al., 1995). The group consisting of male BL6/C57 mice weighing 20 g to 23 g was used in the experiment. The experiment proceeded after experimental animals were accustomed to the conventional environment of 23±2°C and 55±15% humidity for 3 days, in which light was constrained from 8 pm to 8 am, and were given ad libitum access to food and water.

Atopic dermatitis animal models were produced with reference to Hatano et al. (2011), and it is known that TRPV1 is mediated in the developmental mechanism.

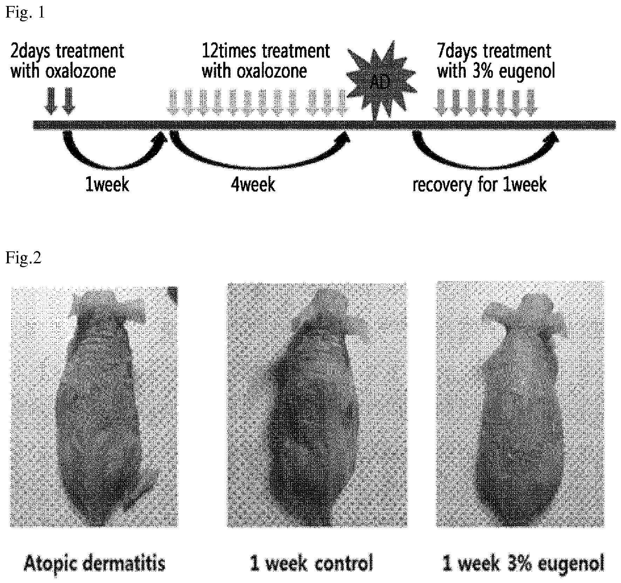

Particularly, balb/c nude mice were used to produce the atopic dermatitis animal models. In the first two days, 50 µL of 5% oxalozone was treated to sensitize the back of the neck of experimental animals, and a week of recovery followed. In the next 4 weeks, 60 µL of 0.5% oxalozone was treated for a total of 12 times to complete atopic dermatitis animal models (Fig. 1).

### Experimental Example 1: Visual inspection of effects of eugenol on improving atopic dermatitis

Atopic dermatitis animal models induced in Example were treated with 200 µL of either base cream not containing eugenol or a 3 wt% eugenol cream at the same time of a day for a week, or untreated, and the effects of a treating 3 wt% eugenol cream on dermal recovery from atopic dermatitis were examined.

As shown in Fig. 2, it was observed that treating eugenol for a week recovered skin to the normal level.

### Experimental Example 2: Histological examination of epidermis and dermis regarding effects of eugenol on improving atopic dermatitis

In order to examine the changes of epidermis and dermis of experimental animals following induction of atopic dermatitis, hematoxylin & eosin (H&E) staining was performed. Epidermis and dermis tissues were separated from experimental animals, fixed using a tissue fixative, 4% paraformaldehyde solution (pH 7.4), and cut into 5 µm to 6 µm fragments after embedding in paraffin following conventional tissue sample manufacturing methods. After tissue samples were removed from paraffin using xylene, the nucleus and cytoplasm were stained with water-containing H&E, and the changes were observed using an optical microscope.

The results from examining the histological changes of epidermis and dermis showed that atopic dermatitis animal models experienced epidermis hyperplasia, and the epidermis thickness thereof was significantly increased, compared to the control group. This suggests that atopic dermatitis animal models experience changes not only at the level of appearance but also at histological level and thus are examined to qualify for atopic dermatitis requirements. When the animal models were allowed to naturally heal (untreated group) or treated with a base cream for a week, both showed recovery at a certain level, but epidermis hyperplasia was still observed (Fig. 3). However, when a 3 wt% eugenol was treated for a week, the animal models showed similar histological results to the control group. This suggests that eugenol exhibits effects of inducing fast recovery of epidermis and dermis in atopic dermatitis animal models

### Experimental Example 3: Antipruritic effects of eugenol in atopic dermatitis animal models

After making atopic dermatitis animal models, the effects of eugenol on pruritus related to atopic dermatitis were observed. Atopic dermatitis animal models produced in Example were treated with 200 µL of a 3wt% eugenol cream, and the control group was treated with a base cream. Voluntary pruritus reactions were observed for an hour, and recovery effects after treating eugenol were compared.

The results showed that atopic dermatitis animal models engaged in a scratching behavior about 40 times in an hour, which is a significantly increase compared to the normal which only engaged in a scratching behavior less than 10 times. However, after treating eugenol to atopic dermatitis animal models, the scratching behavior was similar to that of the normal animals (Fig. 4).

### Experimental Example 4: Effects of eugenol on pruritus via a scratch test

In order to confirm reactions to pruritus-inducing substances such as histamine or serotonin in the present invention, a behavior experiment was conducted. In order to induce scratching, histamine and serotonin which are known to induce itchy sensation in humans were used. Here, histamine or serotonin was diluted with sterilized saline solution. On the day before the experiment, experimental animals were anaesthetized by mixing zolitel and rompun at a ratio of 1:4 and injecting 1 mL/kg thereof, and hair on the nape areas of a 1.5cm × 1.5cm square was removed using a clipper on the nape of experimental animals. On the day of the experiment, a box of 10cm × 20cm × 15cm dimensions was produced and mice were stabilized for 30 minutes therein. Thereafter, 100 µL of solvents or the experimental substance was applied on the back of the neck of mice, and after 30 minutes, 50 µL of pruritus-inducing substances (500 µg/site of histamine or 10 µg/site of serotonin) was injected at the back of the neck of experimental animals via intradermal injection. Next, reactions of experimental animals were video-recorded for 30 minutes, and a scratch test was conducted by counting up and down of a hind paw of an experimental animal to the injected area in the back of the neck as one scratch. Further, eugenol was applied as a cream at 3 different concentrations (1 wt%, 3 wt%, and 10 wt%), reactions to histamine- or serotonin-induced pruritus were examined. Here, the amount of a cream formulation applied was either 100 µL or 200 µL.

The results showed that a cream not containing eugenol did not significantly reduce pruritus induced by histamine (500 µg), but treating 3 wt% eugenol reduced pruritus reactions by at least 40%. Further, an amount-dependent manner was observed, as 10 wt% eugenol increased blocking effects on histamine-dependent pruritus, etc.

Meanwhile, the effects of eugenol on pruritus induced by serotonin (10 µg) were examined. It was confirmed that unlike the histamine-induced pruritus, antipruritic effects were not observed in the group treated with 100 µL of 3 wt% eugenol, and the group treated with 100 µL of 10 wt% eugenol showed blocking of serotonin-induced pruritus. The results suggest that eugenol has effects on either histamine- or serotonin-induced pruritus, but acts more effectively on histamine-induced pruritus.

### Experimental Example 5: Effects of eugenol derivatives on pruritus

As described in Experimental Example 4, in order to induce scratching, experimental animals in the control group were injected with 500 µg/site of histamine at the back of the neck via intradermal injection, and along with the histamine, the experimental group was simultaneously injected with 10 µM isoeugenol (mixture of cis and trans, 82 ng) and 10µM methyleugenol (89 ng) as representative eugenol derivatives, as well as 10µM eugenol (82 ng). Thereafter, the reactions of experimental animals were video-recorded for 30 minutes, and a scratch test was conducted by counting the action of going up and down of a hind paw of an experimental animal to the injected area in the back of the neck as one scratch.

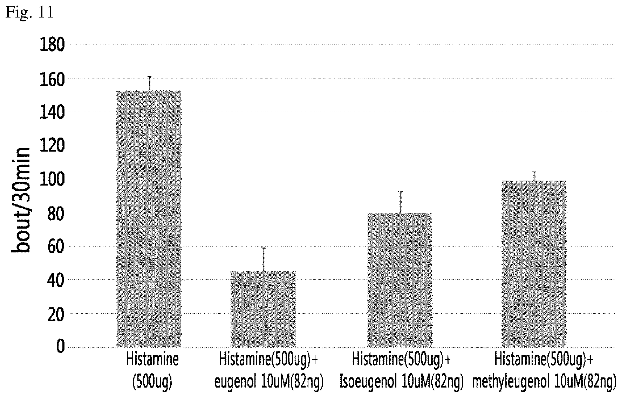

The results showed that while the animal models intracutaneously injected with only histamine scratched about 152 times on average, the animal models (i) simultaneously injected with eugenol scratched about 45 times on average; (ii) injected with isoeugenol scratched about 80 times on average; and (iii) injected with methyleugenol scratched 99 times on average; thereby confirming that simultaneous injection of eugenol and the derivatives thereof significantly reduced the number of scratching (Fig. 11). This suggests that antipruritic effects were exhibited on histamine-induced pruritus not only by eugenol, but also by eugenol derivatives.

### Experimental Example 6: Effects of eugenol on acute mechanical pain reactions and rotarod motor activity

### Experimental Example 6-1: Effects of eugenol on acute mechanical pain reactions

Eugenol is used as anesthetics for dentistry and may exhibit effects of blocking pain by blocking voltage-dependent Na ion channels present in sensory neurons. However, effects of blocking pain by blocking Na ion channels are induced by blocking all the dermal sensory information and thus are non-specific phenomena. Therefore, von-Frey test was conducted to examine whether antipruritic effects are induced by reduced excitation via blocking voltage-dependent Na ion channels of sensory nerves by confirming whether eugenol at a pruritus-effective concentration blocks pain reactions. With reference to Chaplan et al. (1994), hind paws of experimental animals were stimulated with von-Frey filaments (0.02g to 4g), and 50% avoidance reactions were measured. Through this process, avoidance reactions to mechanical stimulations were measured and assessed as a reaction threshold.

The results showed that 3 wt% eugenol did not show a significant difference in mechanical pain using von-Frey. This suggests that 3wt% eugenol is not related to anesthetic effects induced by blocking voltage-dependent Na ion channels.

### Experimental Example 6-2: Effects of eugenol on motor activity

In the present invention, a rotarod test was conducted to examine the effects of eugenol on motor performance ability of the subjects. It was decided to conduct a rotarod test by rotating the drum by gradually increasing the speed from 1 rpm to 40 rpm in 30 seconds, and the time till the fall from the drum was measured. If eugenol exhibits effects on motor ability, the time will be shortened. Further, in order to examine whether pruritus reactions are reduced by reduced motor abilities due to eugenol, motor abilities were examined while varying eugenol concentrations.

The results showed that all eugenol-treated groups (200 µL of 3 wt%, 200 µL of 10 wt%, and 400 µL of 10 wt%) showed similar motor abilities to that of the control group. This suggests that pruritus-effective eugenol concentrations do not reduce motor abilities of a subject and act specifically to inhibit pruritus.

### Experimental Example 7: Effects of eugenol on acute heat pain reactions

In order to examine whether 3 wt% eugenol acts on capsaicin receptors and effects behavioral reactions to heat stimulation, Hargreaves test was conducted. With reference to Hargreaves et al. (1988), heat stimulation by light was delivered to a hind paw of a subject, and the corresponding avoidance reaction time was measured.

The results showed that 3 wt% eugenol significantly increased avoidance reaction in response to heat pain, compared to the base cream which is a cream formulation, which means that the eugenol cream blocks heat pain. Accordingly, this suggests that eugenol acts on histamine-dependent pruritus by blocking capsaicin receptors.

### Experimental Example 8: Duration assessment of antipruritic effects of eugenol cream

In order to examine the duration of antipruritic effects of eugenol, after inducing histamine-induced pruritus, the duration of eugenol effects was examined. At each of an hour, 3 hours, and 5 hours prior to inducing histamine-induced pruritus, 200 µL of a 3wt% eugenol cream was pretreated, and after the corresponding times have passed, histamine-induced pruritus was induced as in Experimental Example 4. Meanwhile, the control groups were treated with the base cream.

As a result, while histamine-induced pruritus animal models engaged in a scratching behavior about 152 times in 30 minutes, the animal models treated with base cream engaged in a scratching behavior about 141 times in 30 minutes, the animal models pretreated with the eugenol cream an hour before inducing histamine-induced pruritus engaged in a scratching behavior about 59 times, which confirmed calming effects in a short period of time. Furthermore, the animal models with 3-hour-prior and 5-hour-prior pretreatments engaged in a scratching behavior about 70 times and 107 times, respectively, thereby confirming pruritus-calming effects lasting up to 5 hours (Fig. 10).

## Claims

1. A pharmaceutical composition for use in the improvement, prevention or treatment of pruritus, comprising a compound represented by Formula 1 or Formula 2, or a pharmaceutically acceptable salt thereof as an active ingredient, 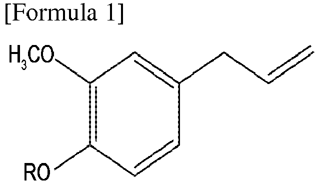 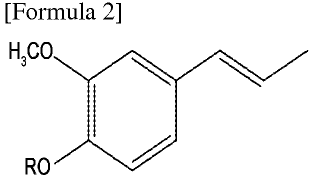 wherein, in the formulae, R is hydrogen or C₁₋₄ alkyl.

2. The composition for use according to claim 1, wherein R in Formula 1 is hydrogen or methyl, and R in Formula 2 is hydrogen.

3. The composition for use according to claim 1, wherein the composition further comprises a pharmaceutically acceptable carrier.

4. The composition for use according to claim 1, wherein the concentration of the compound or the pharmaceutically acceptable salt thereof ranges from 3 wt% to 10 wt%.

5. The composition for use according to claim 1, wherein the compound or the pharmaceutically acceptable salt thereof is **characterized by** blocking capsaicin receptors.

6. A health functional food composition for use in the prevention or improvement of pruritus, comprising a compound represented by Formula 1 or Formula 2, or a pharmaceutically acceptable salt thereof as an active ingredient, 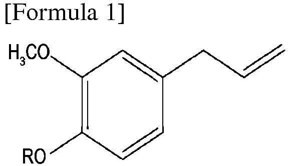 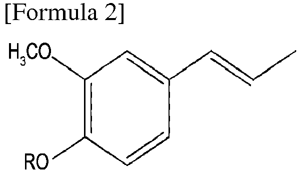 wherein R is hydrogen or C₁₋₄ alkyl in the formulae.

7. A cosmetic composition for use in the prevention or improvement of pruritus, comprising a compound represented by Formula 1 or Formula 2, or a pharmaceutically acceptable salt thereof as an active ingredient, 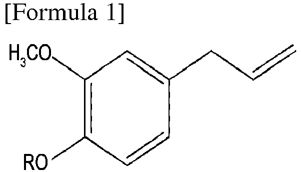 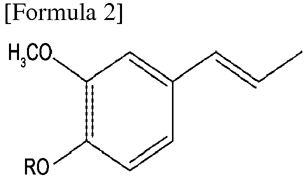 wherein, in the formulae, R is hydrogen or C₁₋₄ alkyl.

8. The composition for use according to claim 6 or 7, wherein R in Formula 1 is hydrogen or methyl, and R in Formula 2 is hydrogen.

9. The composition for use according to claim 6 or 7, wherein the concentration of the compound or the pharmaceutically acceptable salt thereof ranges from 3 wt% to 10 wt%.

10. A transdermal formulation for use in the treatment of pruritus, comprising a composition according to any one of claims 1 to 5.

11. The formulation for use according to claim 10, wherein the transdermal formulation is in the form of ointments, creams, gels, lotions, solutions, emulsions, suspensions, sticks, pastes, liniments, cataplasms, tapes, aerosols, or external powders.

## Patentansprüche

1. Eine pharmazeutische Zusammensetzung zur Verwendung bei der Verbesserung, Vorbeugung oder Behandlung von Juckreiz, umfassend eine Verbindung, dargestellt durch Formel 1 oder Formel 2, oder ein pharmazeutisch annehmbares Salz davon als einen aktiven Bestandteil, 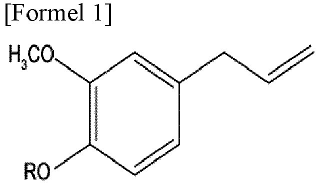 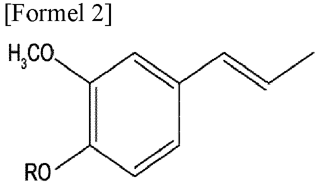 wobei, in den Formeln, R Wasserstoff oder C₁₋₄-Alkyl ist.

2. Die Zusammensetzung zur Verwendung nach Anspruch 1, wobei R in Formel 1 Wasserstoff oder Methyl ist und R in Formel 2 Wasserstoff ist.

3. Die Zusammensetzung zur Verwendung nach Anspruch 1, wobei die Zusammensetzung weiterhin einen pharmazeutisch annehmbaren Träger umfasst.

4. Die Zusammensetzung zur Verwendung nach Anspruch 1, wobei die Konzentration der Verbindung oder des pharmazeutisch annehmbaren Salzes davon im Bereich von 3 Gew.-% bis 10 Gew.-% liegt.

5. Die Zusammensetzung zur Verwendung nach Anspruch 1, wobei die Verbindung oder das pharmazeutisch annehmbare Salz davon durch die Blockierung von Capsaicin-Rezeptoren gekennzeichnet ist.

6. Eine gesundheitsfunktionelle Nahrungsmittelzusammensetzung zur Verwendung bei der Vorbeugung oder Verbesserung von Juckreiz, umfassend eine Verbindung, dargestellt durch Formel 1 oder Formel 2, oder ein pharmazeutisch annehmbares Salz davon als einen aktiven Bestandteil, 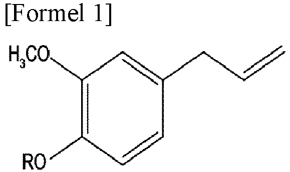 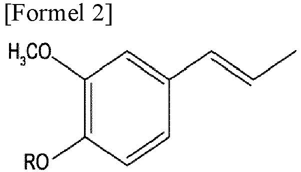 wobei, in den Formeln, R Wasserstoff oder C₁₋₄-Alkyl ist.

7. Eine kosmetische Zusammensetzung zur Verwendung bei der Vorbeugung oder Verbesserung von Juckreiz, umfassend eine Verbindung, dargestellt durch Formel 1 oder Formel 2, oder ein pharmazeutisch annehmbares Salz davon als einen aktiven Bestandteil, 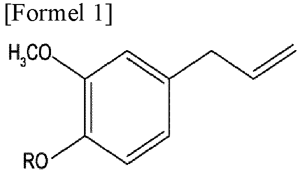 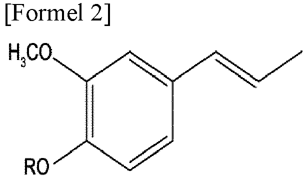 wobei, in den Formeln, R Wasserstoff oder C₁₋₄-Alkyl ist.

8. Die Zusammensetzung zur Verwendung nach Anspruch 6 oder 7, wobei R in Formel 1 Wasserstoff oder Methyl ist und R in Formel 2 Wasserstoff ist.

9. Die Zusammensetzung zur Verwendung nach Anspruch 6 oder 7, wobei die Konzentration der Verbindung oder des pharmazeutisch annehmbaren Salzes davon im Bereich von 3 Gew.-% bis 10 Gew.-% liegt.

10. Eine transdermale Formulierung zur Verwendung bei der Behandlung von Juckreiz, umfassend eine Zusammensetzung nach einem der Ansprüche 1 bis 5.

11. Die Formulierung zur Verwendung nach Anspruch 10, wobei die transdermale Formulierung in Form von Salben, Cremes, Gelen, Lotionen, Lösungen, Emulsionen, Suspensionen, Stäbchen, Pasten, Linimenten, Kataplasmen, Bändern, Aerosolen oder externen Pudern vorliegt.

## Revendications

1. Composition pharmaceutique destinée à être utilisée dans l'atténuation, la prévention ou le traitement d'un prurit, comprenant un composé représenté par la formule 1 ou la formule 2, ou un sel pharmaceutiquement acceptable d'un tel composé, en tant qu'un composant actif, 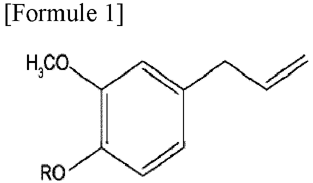 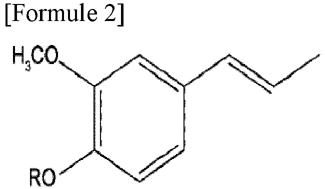 dans laquelle, dans les formules, R est un atome d'hydrogène ou un groupe alkyle en C₁-C₄.

2. Composition destinée à être utilisée selon la revendication 1, dans laquelle R dans la formule 1 est un atome d'hydrogène ou un groupe méthyle, et R dans la formule 2 est un atome d'hydrogène.

3. Composition destinée à être utilisée selon la revendication 1, dans laquelle la composition comprend en outre un véhicule pharmaceutiquement acceptable.

4. Composition destinée à être utilisée selon la revendication 1, dans laquelle la concentration du composé ou du sel pharmaceutiquement acceptable d'un tel composé se situe dans la plage de 3 % en poids à 10 % en poids.

5. Composition destinée à être utilisée selon la revendication 1, dans laquelle le composé ou le sel pharmaceutiquement acceptable d'un tel composé est **caractérisé par** le blocage des récepteurs de la capsaïcine.

6. Composition d'aliment fonctionnel diététique destinée à être utilisée dans la prévention ou l'atténuation d'un prurit, comprenant un composé représenté par la formule 1 ou la formule 2, ou un sel pharmaceutiquement acceptable d'un tel composé, en tant qu'un composant actif, 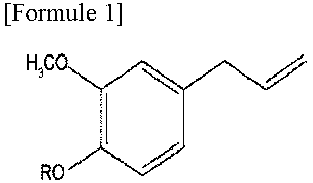 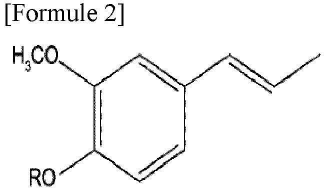 dans laquelle, dans les formules, R est un atome d'hydrogène ou un groupe alkyle en C₁-C₄.

7. Composition cosmétique destinée à être utilisée dans la prévention ou l'atténuation d'un prurit, comprenant un composé représenté par la formule 1 ou la formule 2, ou un sel pharmaceutiquement acceptable d'un tel composé, en tant qu'un composant actif, 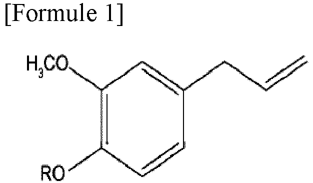 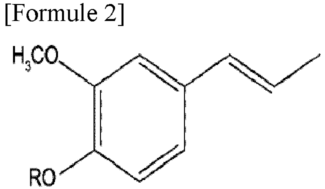 dans laquelle, dans les formules, R est un atome d'hydrogène ou un groupe alkyle en C₁-C₄.

8. Composition destinée à être utilisée selon la revendication 6 ou 7, dans laquelle R dans la formule 1 est un atome d'hydrogène ou un groupe méthyle, et R dans la formule 2 est un atome d'hydrogène.

9. Composition destinée à être utilisée selon la revendication 6 ou 7, dans laquelle la concentration du composé ou du sel pharmaceutiquement acceptable d'un tel composé se situe dans la plage de 3 % en poids à 10 % en poids.

10. Formulation transdermique destinée à être utilisée dans le traitement d'un prurit, comprenant une composition selon l'une quelconque des revendications 1 à 5.

11. Formulation destinée à être utilisée selon la revendication 10, dans laquelle la formulation transdermique est sous la forme de pommades, crèmes, gels, lotions, solutions, émulsions, suspensions, bâtons, pâtes, liniments, cataplasmes, bandes, aérosols, ou poudres à usage externe.
